# EUROPEAN PATENT APPLICATION

(11) **EP 4 685 746 A2**
(43) Date of publication of application: **28.01.2026**
(21) Application number: 25222669.1
(22) Date of filing: 05.09.2023
(51) Int. Cl.: G06T 7/68

(54) **TECHNIQUE FOR DETERMINING AT LEAST ONE PREDICTION IMAGE BASED ON AT LEAST ONE MEDICAL IMAGE OF A PATIENT**

(62) Divisional of application: 23195265.6
(71) Applicant: Stryker European Operations Limited, Carrigtwohill, Co. Cork T45 HX08 (IE)
(72) Inventor: Weede, Oliver, 79110 Freiburg (DE); Schöpp, Hans, 79110 Freiburg (DE)
(74) Representative: Schott, Jakob Valentin

(57) **Abstract**

A method for determining at least one prediction image based on at least one medical image of a patient is provided. The method comprises: obtaining image data including at least one medical image comprising representations of a plurality of bones of a patient's body in a first body posture; obtaining alignment data indicative of an alignment of at least two of the plurality of bones of the patient's body in a second body posture differing from the first body posture; determining, based on the image data and the alignment data, at least one prediction image comprising representations of the plurality of bones of the patient's body in the first body posture, wherein determining the at least one prediction image comprises modifying the at least one medical image based on the alignment data such that poses of the representations of the at least two bones after the modification comply with the alignment of the at least two bones indicated by the alignment data; and triggering display of one or more of the at least one prediction image and/or of information derived from one or more of the at least one prediction image. A corresponding system, computer program and carrier are also provided.

## Description

### Technical Field

The present disclosure generally relates to a method for determining at least one prediction image based on at least one medical image of a patient. A system, a computer program and a carrier are also provided.

### Background

In many surgical scenarios, a patient must be in a predefined body posture (e.g., in a supine position) to enable surgery, the predefined body posture generally enabling a surgeon to reach anatomical structures of interest. The surgeon may then conduct a surgical procedure to permanently change relative poses between at least two bones of the patient. To support the surgeon in this step, current systems provide the surgeon with a navigation image in which current poses of tracked bones of the patient in the predefined body posture are indicated.

The predefined body posture during surgery may deviate from body postures that the patient is regularly in when no surgery is performed (e.g., a standing or sitting position). In current solutions, a surgeon has to rely on his experience to predict a surgical outcome, in particular to predict poses of the patient's bones after surgery when the patient is no longer in the predefined body posture.

For example, spinal procedures for fusing vertebrae are usually carried out when the patient is lying down. Fusing two vertebrae together with an intended relative angle therebetween and with the patient being in the lying posture effects a similar angular change between these fused vertebrae when the patient is subsequently in the standing posture. The effect of this angular change on the overall spinal shape with the patient in the standing posture on the other hand may be hard to predict by a surgeon.

### Summary

There is a need for a technique that solves one or more of the aforementioned or other problems.

According to a first aspect, a method for determining at least one prediction image based on at least one medical image of a patient is provided. The method is performed by at least one processor and comprises obtaining image data including at least one medical image comprising representations of a plurality of bones of a patient's body in a first body posture. The method further comprises obtaining alignment data indicative of an alignment of at least two of the plurality of bones of the patient's body in a second body posture differing from the first body posture. The method comprises determining, based on the image data and the alignment data, at least one prediction image comprising representations of the plurality of bones of the patient's body in the first body posture. Determining the at least one prediction image comprises modifying the at least one medical image based on the alignment data such that poses of the representations of the at least two bones after the modification comply with the alignment of the at least two bones indicated by the alignment data. The method further comprises triggering display of one or more of the at least one prediction image and/or of information derived from one or more of the at least one prediction image.

This method may be particularly suitable for informing a surgeon on poses of (e.g., the plurality of) the patient's bones in the first body posture based on the alignment of (e.g., the at least two of) the patient's bones in the second body posture.

The at least two bones may be in a weight bearing state when the patient's body is in the first body posture. Alternatively, or in addition, the at least two bones may be in a non-weight bearing state when the patient's body is in the second body posture.

The first body posture may correspond to a standing posture. Alternatively, or in addition, the second body posture may correspond to a lying posture.

Modifying the at least one medical image may comprise dividing the at least one medical image into a plurality of image portions and changing a pose of at least one of the plurality of image portions relative to one or more other ones of the plurality of image portions.

Each of the plurality of image portions may contain a representation of a respective different one of the at least two of the plurality of bones.

The at least one medical image may be divided at least along a line or plane associated with a surface of one of the at least two bones.

The at least two bones may correspond to different vertebrae.

The line or plane may be associated with an endplate of one of the different vertebrae facing toward another one of the different vertebrae.

Determining the at least one prediction image may comprise applying a rotational transformation to the modified at least one medical image to adjust at least one spinal balance parameter of the patient's spine derived from the modified at least one medical image.

The method may comprise obtaining patient-specific information indicative of one or more of an age of the patient, a gender of the patient, a height of the patient, a weight of the patient and/or a Body Mass Index, BMI, of the patient. The rotational transformation may be determined based on the patient-specific information.

The rotational transformation may be determined such that by applying the rotational transformation to the modified at least one medical image, the modified at least one medical image is rotated around a femoral rotation axis. The femoral rotation axis may intersect a center point of a representation of a head of one of the patient's femoral bones and, optionally, a center point of a representation of a head of another one of the patient's femoral bones.

The method may further comprise deriving, as the information, at least one spinal balance parameter from one or more of the at least one prediction image. Deriving the at least one spinal balance parameter may comprise identifying at least one anatomical landmark in the one or more of the at least one prediction image and determining the at least one spinal balance parameter based on a pose of the identified at least one anatomical landmark.

The at least one spinal balance parameter may comprise one or more of the following: Pelvic Incidence, PI; Pelvic Tilt, PT; T1 pelvic angle, TPA; Cobb angle; Sacral Slope, SS; Sagittal Vertical Axis, SVA.

The at least one medical image may comprise a pre-operative lateral, LAT, X-ray image and/or an anterior-posterior, AP, X-ray image.

The alignment of the at least two bones may comprise at least two poses selected from (i) a tracked pose of one or more of the at least two bones of the patient's body in the second body posture and (ii) an imaged pose of one or more of the at least two bones of the patient's body in the second body posture.

The method may further comprise obtaining at least one second medical image comprising representations of the at least two bones of the patient's body in the second body posture. The method may comprise obtaining tracking data indicative of a tracked pose of a surgical instrument. The method may comprise triggering display of a navigation view that is based on one or more of the at least one second medical image and based on the tracking data and visualizes a tracked pose of the surgical instrument relative to the at least two bones of the patient's body in the second body posture.

According to a second aspect, a system is provided. The system comprises at least one processor configured to perform the method according to the first aspect. The at least one processor may be configured to: obtain image data including at least one medical image comprising representations of a plurality of bones of a patient's body in a first body posture; obtain alignment data indicative of an alignment of at least two of the plurality of bones of the patient's body in a second body posture differing from the first body posture; determine, based on the image data and the alignment data, at least one prediction image comprising representations of the plurality of bones of the patient's body in the first body posture, wherein determining the at least one prediction image comprises modifying the at least one medical image based on the alignment data such that poses of the representations of the at least two bones after the modification comply with the alignment of the at least two bones indicated by the alignment data; and trigger display of one or more of the at least one prediction image and/or of information derived from one or more of the at least one prediction image.

The system may further comprise at least one of the following entities: a tracking system configured to acquire a tracked pose of one or more of the at least two bones; one or more trackers each having a fixed spatial relationship to a respective one of the at least two bones; an image acquisition system configured to acquire a (e.g., the and/or the second) medical image of one or more (e.g., the plurality of and/or the at least two of the plurality of) bones of the patient's body; a display unit configured to display the prediction image and/or the information derived from the prediction image upon being triggered to display said image and/or information.

According to a third aspect, a computer program is provided. The computer program comprises instructions which, when executed on at least one processor, cause the at least one processor to perform the method according to the first aspect. The computer program may comprise instructions which, when executed on the at least one processor, cause the at least one processor to: obtain image data including at least one medical image comprising representations of a plurality of bones of a patient's body in a first body posture; obtain alignment data indicative of an alignment of at least two of the plurality of bones of the patient's body in a second body posture differing from the first body posture; determine, based on the image data and the alignment data, at least one prediction image comprising representations of the plurality of bones of the patient's body in the first body posture, wherein determining the at least one prediction image comprises modifying the at least one medical image based on the alignment data such that poses of the representations of the at least two bones after the modification comply with the alignment of the at least two bones indicated by the alignment data; and trigger display of one or more of the at least one prediction image and/or of information derived from one or more of the at least one prediction image. The computer program may be carried by at least one carrier such as a non-transitory computer-readable storage medium. The system of the second aspect may comprise said non-transitory computer readable medium, e.g., as part of a computer memory.

According to a fourth aspect, a carrier is provided. The carrier carries the computer program according to the third aspect. The carrier may be a data stream. The carrier may be a non-transitory computer-readable storage medium.

### Brief Description of the Drawings

Further details, advantages and aspects of the present disclosure will become apparent from the following embodiments taken in conjunction with the drawings, wherein:
- Fig. 1: shows an embodiment of a system in accordance with the present disclosure;
- Fig. 2: shows a flowchart of a method embodiment in accordance with the present disclosure;
- Figs. 3a-3b: illustrate spinal balance parameters in accordance with the present disclosure;
- Fig. 4a: illustrates an exemplary medical image in accordance with the present disclosure;
- Fig. 4b: illustrates an exemplary second medical image in accordance with the present disclosure;
- Fig. 5a: illustrates a navigation view in accordance with the present disclosure;
- Fig. 5b: illustrates an exemplary modified medical image that may be used as the prediction image in accordance with the present disclosure; and
- Fig. 6: illustrates a rotation of a modified medical image in accordance with the present disclosure.

### Detailed Description

In the following description, exemplary embodiments will be explained with reference to the drawings. The same reference numerals will be used to denote the same or similar structural features, unless indicated otherwise.

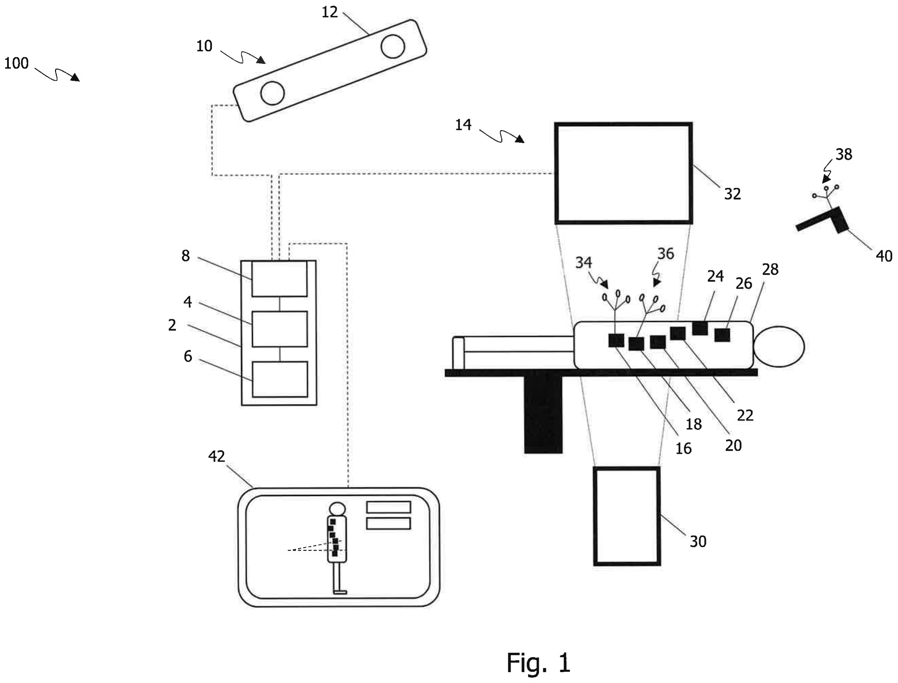

Fig. 1 shows a system 100 in accordance with the present disclosure. The system 100 may be a surgical navigation system.

The system 100 comprises a processing apparatus 2 including at least one processor 4. The at least one processor 4 is communicatively coupled to a memory 6, which is one example of a non-transitory computer-readable storage medium. The at least one processor 4 may be further communicatively coupled to an interface 8 of the apparatus 2. The apparatus 2 may be implemented as a spatially distributed processing apparatus 2, for example by implementing the at least one processor 4, the memory 6 and/or the interface on different servers (e.g., as virtual processor(s), virtual memory or memories). In other words, at least parts of the apparatus 2 may be realized by a cloud computing platform. Alternatively, the apparatus 2 may be implemented as a local processing apparatus 2 that is for example arranged in a scanning room or an operating room of a hospital.

The system 100 further comprises a tracking system 10. In the illustrated example, the tracking system 10 is an optical tracking system comprising a stereo camera 12. The tracking system 10 may be communicatively coupled to the at least one processor 4 via the interface 8 and may be configured provide the at least one processor 4 with tracking data indicative of tracked poses of trackers. The term "pose" as used herein is to be understood as meaning "at least one of a position and an orientation".

The system 100 further comprises an image acquisition system 14 configured to acquire a medical image of one or more bones 16-26 of the patient's body 28. The image acquisition system 14 may be configured to provide image data including the acquired medical image(s) to the at least one processor 4. To this end, the medical image acquisition system 14 may be communicatively coupled to the at least one processor 4 via the interface 8. For example, the medical image acquisition system 14 may be configured to acquire a computed tomography, CT, image of the patient's body 28 as the medical image and comprise a C-arm having an X-ray source 30 and an X-ray detector 32. The image data may be provided as a single dataset or as multiple datasets. For example, each of a plurality of X-ray images acquired by the medical image acquisition system 14 may be separately transmitted to the at least one processor 4 or as a bundle of images.

The system 100 further comprises one or more trackers 34-38. Each of the trackers 34, 36 are fixedly positioned relative to a respective bone 16, 18 of the patient's body 28, and may thus be referred to as bone trackers. It is noted that the step of arranging the trackers 34, 36 accordingly is not part of the technique disclosed herein. The tracker 38 is attached to a medical instrument 40 handled by a surgeon or a surgical robot, and may thus be referred to as instrument tracker. Each of the trackers 34-38 can be tracked by the tracking system 10, which means the tracking system 10 can determine the pose of each of the trackers 34-38 in a (e.g., real-world) tracking coordinate system. The (e.g., origin and orientation of the) tracking coordinate system may be defined by one of the trackers, for example by the patient tracker 34. The tracking system 10 may be configured to provide the at least one processor 4 with information indicative of the tracked poses of the trackers 34-38, for example in the tracking coordinate system. The tracking system 10 may be configured to acquire a tracked pose of one or more of the bones 16, 18 by localizing one or more of the trackers 34, 36 in the tracking coordinate system. The trackers 34, 36 may each have a predefined spatial relationship relative to the respective bones 16, 18. In the illustrated example, each of the trackers 34-38 comprises a plurality of optical tracking markers detectable by the stereo camera 12, but the present disclosure is not limited thereto. For example, instead of an optical tracking system, an electromagnetic tracking system may be employed.

The system 100 further comprises a display unit 42. The display unit 42 may be communicatively coupled to the at least one processor 4 via the interface 8. One or more images and/or information (e.g., in the form of text, numbers and/or symbols) may be displayed by the display unit 38 when being triggered accordingly by the at least one processor 4.

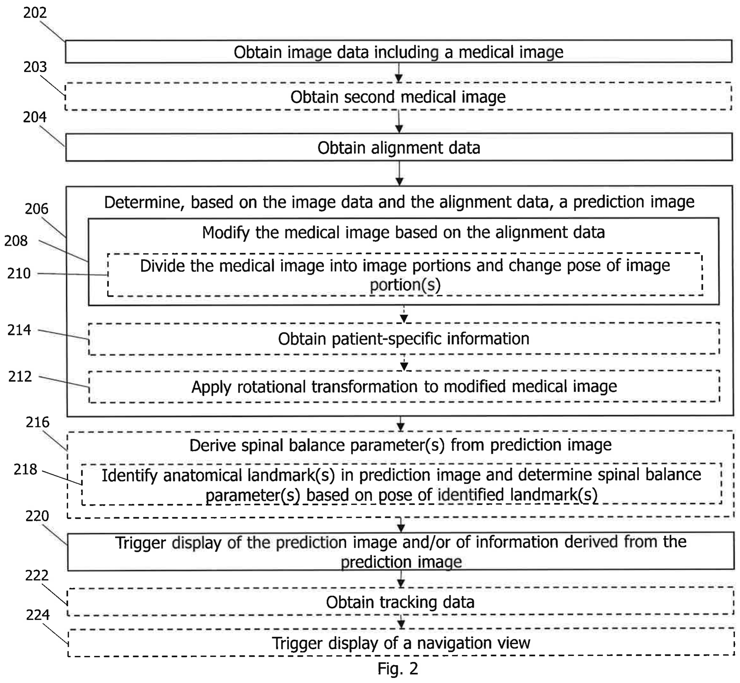

Fig. 2 shows a method in accordance with the present disclosure. The method may be performed by the at least one processor 4. The memory 6 may store a computer program comprising instructions which, when executed by the at least one processor 4, cause the at least one processor 4 to perform the corresponding method.

In step 202, image data including at least one medical image is obtained.

The at least one medical image comprises representations of a plurality of bones 16-26 (e.g., three or more bones) of a patient's body 28 in a first body posture.

The image data may be obtained from the medical image acquisition system 14. The at least one medical image consists of a single medical image or of a plurality of medical images. The at least one medical image may comprise or be a two-dimensional image. The at least one medical image may comprise or be a three-dimensional image. The at least one medical image may comprise or be an X-ray image, a computed tomography, CT, image, or a magnetic resonance, MR, image. For example, each of the at least one medical image is a two-dimensional X-ray image. Each of the at least one medical image obtained in step 202 may be a pre-operative image. The representations of the plurality of bones 16-26 comprised in the at least one medical image may be referred to as image representations of the bones 16-26 or as depictions of the bones 16-26. Each of the at least one medical image may be registered relative to one another and/or registered to a common coordinate system.

In one specific variant, the at least one medical image obtained in step 202 comprises a pre-operative lateral, LAT, and/or a pre-operative anterior-posterior, AP, (e.g., X-ray) image.

The plurality of bones 16-26 may be a weight bearing state when the patient's body 28 is in the first body posture. The plurality of bones 16-26 may be configured to bear a weight of the patient's body 28 when the patient's body 28 is in the first body posture. The plurality of bones 16-26 may lie within a same anatomical load path. The plurality of bones 16-26 may be configured to transfer a (e.g., gravitational) force from one to another when the patient's body is in the first body posture. The plurality of bones 16-26 may form one or more joints (e.g., a vertebral joint, a hip joint, a knee joint and/or an ankle joint). The plurality of bones 16-26 may comprise one or more bones from the patient's spine (e.g., including the pelvis), one or more bones from one or both legs of the patient and/or one or more bones from one or both feet or ankles of the patient.

The first body posture may correspond to a standing posture. In this case, the medical (e.g., X-ray) image may have been be acquired while the patient was standing upright. The standing posture may be a normalized standing posture. Such a standing posture may deviate from a relaxed standing posture of the patient. In the normalized standing posture, each arm of the patient may be positioned relative to the patient's torso in a predefined pose.

In optional step 203, at least one second medical image is obtained.

The at least one second medical image comprises representations of at least two bones (e.g., 16, 18) of the plurality of bones 16-26 of the patient's body 28 in a second body posture differing from the first body posture.

The at least one second medical image may be obtained from the medical image acquisition system 14. The at least one second medical image may be included in the image data obtained in step 202. The at least one second medical image consists of a single second medical image or of a plurality of second medical images. The at least one second medical image may comprise or be a two-dimensional image. The at least one second medical image may comprise or be a three-dimensional image. The at least one second medical image may comprise or be an X-ray image, a computed tomography, CT, image, or a magnetic resonance, MR, image. Each of the at least one second medical image obtained in step 203 may be an intraoperative image. The representations of the at least two bones (e.g., 16, 18) comprised in the at least one second medical image may be referred to as image representations of the at least two bones or as depictions of the at least two bones. Each of the at least one second medical image may be registered relative to one another and/or registered to a common coordinate system. The at least one second medical image may represent a same viewing direction of the patient's body 28 (e.g., LAT or AP) as the at least one medical image. In case the at least one medical image consists of a plurality of medical images representing different viewing directions, the at least one second medical image may comprise or consist of a plurality of second medical images representing these different viewing directions.

In one specific variant, the at least one second medical image obtained in step 203 comprises an intraoperative lateral, LAT, and/or an intraoperative anterior-posterior, AP, (e.g., X-ray) image. In another variant, the second medical image data comprises or is an intraoperative CT scan.

The first body posture may differ from the second body posture in a weight bearing state of the at least two (e.g., 16, 18) of the plurality of bones 16-26.

The at least two bones 16, 18 may be a in weight bearing state when the patient's body 28 is in the first body posture. The at least two bones 16, 18 may be configured to bear a weight of the patient's body 28 when the patient's body 28 is in the first body posture. The at least two bones 16, 18 may lie within a same anatomical load path. The at least two bones 16, 18 may be configured to transfer a (e.g., gravitational) force from one to another when the patient's body is in the first body posture. The at least two bones 16, 18 may form one or more joints (e.g., a vertebral joint, a hip joint, a knee joint and/or an ankle joint). The at least two bones 16, 18 may comprise one or more bones from the patient's spine (e.g., including the pelvis), one or more bones from one or both legs of the patient and/or one or more bones from one or both feet or ankles of the patient.

The at least two bones 16, 18 may be in a non-weight bearing state when the patient's body 28 is in the second body posture. The at least two bones 16, 18 may be configured to be (e.g., essentially) free of a weight of the patient's body 28 when the patient's body 28 is in the second body posture. The at least two bones 16, 18 may be under different (e.g., less) load (e.g., stress and/or compression) when the patient's body 28 is in the second body posture compared with when the patient's body 28 is in the first body posture. The at least two bones 16-26 may be configured to not transfer a (e.g., gravitational) force from one to another when the patient's body is in the second body posture.

As mentioned above, the first body posture may correspond to a standing posture. On the other hand, the second body posture may correspond to a lying posture. The patient may be lying on his front or back when that patient's body 28 is in the second body posture. The second body posture may correspond to a prone position or a supine position of the patient's body. That is, the at least one medical image may have been acquired while the patient was standing upright, whereas the at least one second medical (e.g., X-ray) image may have been be acquired while the patient was lying down.

In step 204, alignment data is obtained.

The alignment data is indicative of an alignment of (e.g., the) at least two (e.g., 16, 18) of the plurality of bones 16-26 of the patient's body 28 in the second body posture which differs from the first body posture. The alignment may comprise (e.g., absolute) poses of the at least two of the plurality of bones (e.g., in a common coordinate system such as the tracking coordinate system of the tracking system 10) and/or a relative pose of the at least two of the plurality of bones (e.g., with the patient's body 28 being in the second body posture).

The alignment of the at least two bones may comprise at least two poses selected from (i) a tracked pose of one or more of the at least two bones of the patient's body in the second body posture and (ii) an imaged pose of one or more of the at least two bones of the patient's body in the second body posture. The tracked pose of the one or more of the at least two bones may be obtained by tracking the trackers 16, 18. The tracked pose of the one or more of the at least two bones may be obtained from the tracking system 10. The imaged pose of the one or more of the at least two bones may be obtained by localizing the respective bone(s) in medical image data of the patient's body 18, for example in one or more of the at least one second medical image. The imaged pose of the one or more of the at least two bones may be obtained based on a segmentation of the respective bone(s) in one or more of the at least one second medical image. The obtained pose of a first of the at least two bones may be a tracked pose and the obtained pose of a second of the at least two bones may be an imaged pose. Alternatively, both the first and second pose may be tracked poses or imaged poses.

In step 206, based on the image data and the alignment data, at least one prediction image is determined.

Generally speaking, the at least one prediction image is suitable for informing a surgeon on poses of a patient's bones in the first body posture based on the alignment of the patient's bones in the second body posture. The at least one prediction image comprises representations (e.g., image representations or depictions) of the plurality of bones 16-26 of the patient's body 28 in the first body posture. One may say that the at least one prediction image represents a prediction of poses the plurality of bones 16-26 (e.g., three or more bones) in the first body posture based on the alignment of (e.g., only) the at least two bones in the second body posture as indicated by the alignment data.

Determining the at least one prediction image comprises, at step 208, modifying the at least one medical image based on the alignment data such that poses of the representations of the at least two bones (e.g., 16, 18) after the modification comply with the alignment of the at least two bones indicated by the alignment data. The representations of the at least two bones may be similar in the at least one medical image as in the prediction image, while their relative poses may be different.

In case the at least one medical image consists of a plurality of medical images, each of these medical images may be modified to determine a respective prediction image. In case these medical images differ in the viewing directions of the patient's body, the plurality of determined prediction images may similarly differ in the viewing directions. For example, in case the at least one medical image comprises an AP X-ray image and a LAT X-ray image, a first prediction image may show an anterior-posterior view whereas a second prediction image may show a lateral view. It is also possible to determine the at least one prediction image by modifying only a subset of the plurality of medical images, i.e., determining prediction images only for some of the medical images. In the last example, only the first prediction image may be determined, thereby neglecting the LAT X-ray image.

As indicated in Fig. 2, modifying the at least one medical image may comprise, in optional step 210, dividing the at least one medical image into a plurality of image portions, and changing a pose of at least one of the plurality of image portions relative to one or more other ones of the plurality of image portions. The pose of the at least one image portion may be changed such that the poses of the at least two of the plurality of bones correspond to the poses indicated by the alignment data obtained in step 204.

Each of the plurality of image portions may contain a representation of a respective different one of the at least two of the plurality of bones. Multiple of (e.g., the at least two of) the plurality of bones may be comprised in a same image portion.

The at least one medical image may be divided at least along a line or plane associated with (e.g., a surface, center or outline) of one of the at least two bones. Each of the plurality of image portions may be defined by a portion-specific bone selected from the two or more bones.

The at least two bones may correspond to different vertebrae. The line or plane may be associated with (e.g., approximate) an (e.g., cranial or caudal) endplate of one of the different vertebrae (e.g., facing toward another one of the different vertebrae corresponding to another one of the at least two bones).

The at least one prediction image may be determined as a mosaic of the plurality of image portions having relative poses defined by the alignment data. Overlapping parts of the image portions forming the at least one prediction image may be averaged in image intensity, cropped or processed otherwise. Non-overlapping parts of the image portions may be maintained without further adjustment.

The modified at least one medical image may be used as the at least one prediction image and/or triggered to be displayed.

In optional step 212, a rotational transformation may be (e.g., automatically) applied to the modified at least one medical image.

The rotational transformation may be applied to adjust at least one spinal balance parameter (e.g., Pelvic Incidence, PI and/or Pelvic Tilt, PT) of the patient's spine derived from the modified at least one medical image. The rotational transformation may be applied responsive to determining that the at least one spinal balance parameter of the patient's spine derived from the modified at least one medical image falls outside a predefined normative range (e.g., associated with patient-specific information as described hereinbelow). The PT may be assumed to be constant and calculated before the start of an intervention, but it may alternatively be recalculated by balancing PT on the one hand with SVA or TPA on the other hand (e.g., to make sure both parameters are as close as possible to an ideal value).

A spinal balance parameter as understood herein may comprise a pelvic parameter and/or a global balancing parameter. The at least one spinal balance parameter derived from the modified at least one medical image may comprise one or more of the following: Pelvic Incidence, PI; Pelvic Tilt, PT; T1 pelvic angle, TPA; axial Cobb angle; coronal Cobb angle; sagittal Cobb angle; Sacral Slope, SS; Sagittal Vertical Axis, SVA.

Deriving the at least one spinal balance parameter from the modified at least one medical image may comprise identifying at least one anatomical landmark in the modified at least one medical image and determining the at least one spinal balance parameter based on a pose of the identified at least one anatomical landmark. The at least one anatomical landmark may include one or more predefined portions of the patient's body, for example one or more sections of one of the patient's bones. The at least one anatomical landmark may include at least one of: one or more heads of the patient's femoral bones, the S1 endplate (e.g., including the most posterosuperior corner of the S1), the midpoint of a vertebra such as the seventh cervical vertebra C7, endplates of (e.g., other) vertebrae of interest, landmarks for registration (e.g., postero- and anterosuperior points of vertebral endplates).

The rotational transformation may be determined such that by applying the rotational transformation to the modified at least one medical image, the modified at least one medical image is rotated around a femoral rotation axis or hip axis. The femoral rotation axis may intersect a center of a representation of a head of one of the patient's femoral bones (e.g., in the modified at least one medical image) and optionally a center of a head of another one of the patient's femoral bones. In step 212, relative poses of image portions may remain the same. In other words, the overall modified at least one image may be rotated, not only a subset of the plurality of image portions.

After having applied the rotational transformation, the rotated modified at least one medical image may be used as the at least one prediction image and/or triggered to be displayed.

As indicated in Fig. 2, in optional step 214, patient-specific information may be obtained in a step preceding step 212.

The patient-specific information may be indicative of one or more of an age of the patient, a gender of the patient, a height of the patient, a weight of the patient and/or a Body Mass Index, BMI, of the patient. The rotational transformation applied in optional step 212 may be based on the patient-specific information. For example, the at least one spinal balance parameter derived from the modified at least one medical image may be adjusted by applying the rotational transformation to the modified at least one medical image such that the at least one spinal balance parameter corresponds to a predefined spinal balance parameter associated with the patient-specific information and/or falls into a (e.g., normative and/or predefined) range of the spinal balance parameter associated with the patient-specific information. The amount of rotation applied by the rotational transformation may thus vary depending on the patient-specific information. To simulate Pelivc Tilt in the upright posture, the rotational transformation may be calculated so that at least one of the spinal balance parameters (e.g., TPA), falls within a (e.g., normative and/or predefined) range of spinal balance parameters associated with the patient-specific information (e.g., a normative range of TPA). The method may comprise determining said predefined spinal balance parameter or said range based on the patient-specific information obtained in step 214 and, optionally, further based on one or more spinal balance parameters derived from the modified at least one medical image.

In optional step 216, at least one spinal balance parameter is derived from one or more of the at least one prediction image.

The at least one spinal balance parameter may be associated with a risk of mechanical failure of a spinal implant. The at least one spinal balance parameter may include a health-related factor associated with a post-operative quality of life of the patient. The at least one spinal balance parameter derived from the one or more of the at least one prediction image may comprise one or more of the following: Pelvic Incidence, PI; Pelvic Tilt, PT; T1 pelvic angle, TPA; one or more Cobb angles (e.g., of the vertebrae 16, 18); Sacral Slope, SS; Sagittal Vertical Axis, SVA. The at least one spinal balance parameter may include a global balancing parameter, such as the so-called Global Alignment and Proportion (GAP) score. The global balancing parameter may be determined based on one or more of the following: Pelvic Incidence, PI; Pelvic Tilt, PT; T1 pelvic angle, TPA; one or more Cobb angles (e.g., of the vertebrae 16, 18); Sacral Slope, SS; Sagittal Vertical Axis, SVA.

Deriving the at least one spinal balance parameter from the one or more of the at least one prediction image may comprise, at 218, identifying at least one anatomical landmark (e.g., the vertebra C7, the pelvis and/or a head of the femur) in the one or more of the at least one prediction image and determining the at least one spinal balance parameter based on a pose of the identified at least one anatomical landmark in the one or more of the at least one prediction image.

In step 220, display of one or more of the at least one prediction image and/or of information derived from the one or more of the at least one prediction image is triggered.

The one or more of the at least one prediction image and/or the information derived therefrom may be triggered to be displayed on the display unit 42. The at least one spinal balance parameter derived in optional step 216 may be used as part of the information derived from the one or more of the at least one prediction image and may be triggered to be displayed in step 220.

In optional step 222, tracking data is obtained.

The tracking data is indicative of a tracked pose of a surgical instrument (e.g., 40) and may be obtained from the tracking system 10. The tracked pose may be given in a same coordinate system (e.g., the tracking coordinate system) as the poses of the at least two bones 16, 18 indicated by the alignment data. The at least one second medical image from which the poses of the at least two bones may be derived as imaged poses may be registered relative to the tracking coordinate system using a suitable image registration technique as known to those skilled in the art.

In optional step 224, a navigation view is triggered to be displayed.

The navigation view is based on the at least one second medical image and the tracking data and visualizes a tracked pose of the surgical instrument relative to the at least two bones of the patient's body in the second body posture. As indicated above, the tracking coordinate system of the tracking system 10 may be registered to the at least one second medical image. The navigation view may include a visualization of one or more of the at least one second medical image and an overlay of a symbol (e.g., a trajectory, cross-hairs or the like) representing a tracked pose of the surgical instrument. The navigation view may be triggered to be displayed on the display unit 42. This may allow a surgeon to navigate the surgical instrument relative to the at least two bones 16, 18.

The navigation view may be triggered to be displayed simultaneously with the one or more of the at least one prediction image and/or the information derived therefrom.

For example, the information derived from the one or more of the at least one prediction image may be overlaid in the navigation view. This may allow a surgeon not only to navigate the surgical instrument, but also inform the surgeon on a predicted outcome in case the at least two bones are fixed relative to one another in their current poses as indicated by the alignment data.

Steps of the method of Fig. 2 may be repeated twice or more. For example, alignment data indicating the poses of the at least two bones 16, 18 at a subsequent point in time may be obtained by repeating step 204. It may also be possible that the alignment data obtained in step 204 already indicates poses of the at least two bones for multiple points in time. The steps 206, 208 and 220, and optionally one or more of steps 210-218, 222, 224 may then be performed based on the poses of the at least two bones 16, 18 at the subsequent point in time (e.g., a most recent point in time). In other words, the at least one prediction image may be updated (e.g., periodically or responsive to a change of the poses of one or more of the at least two bones 16, 18).

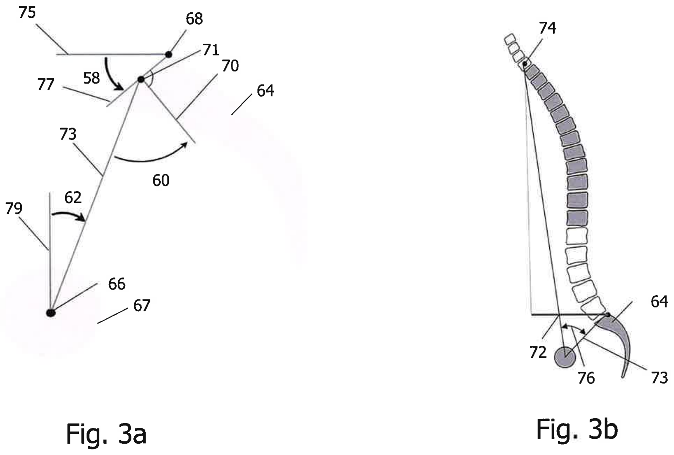

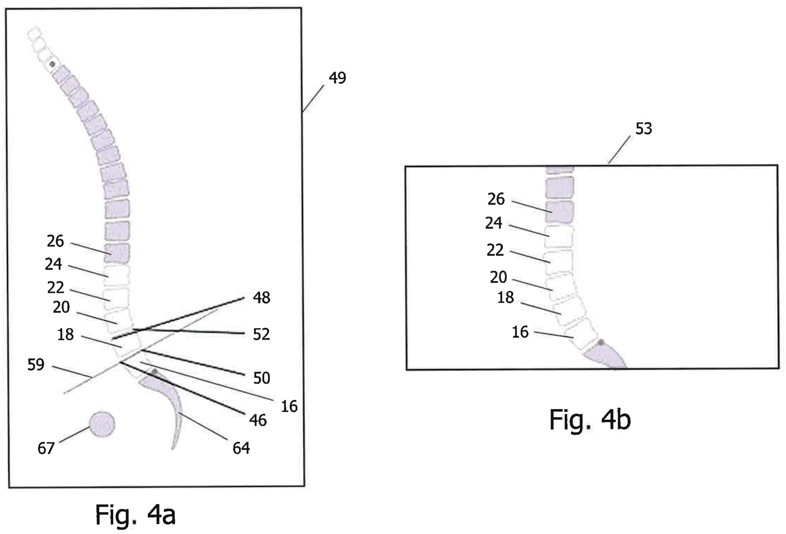

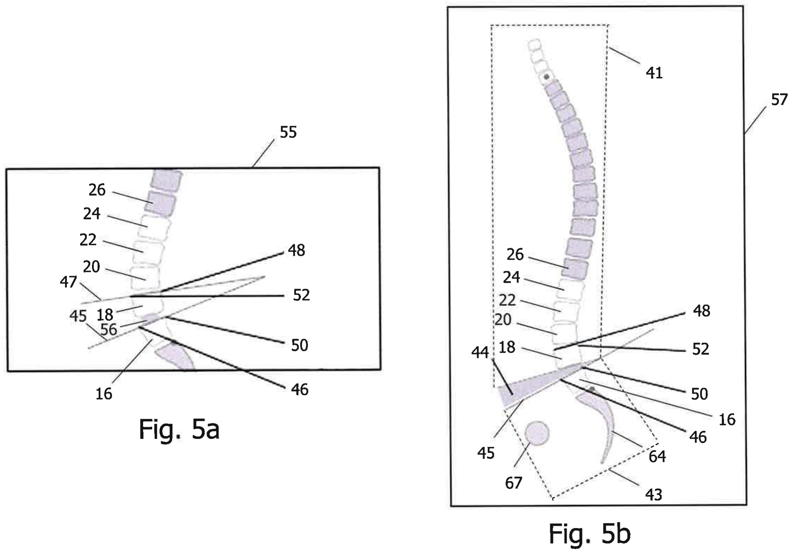

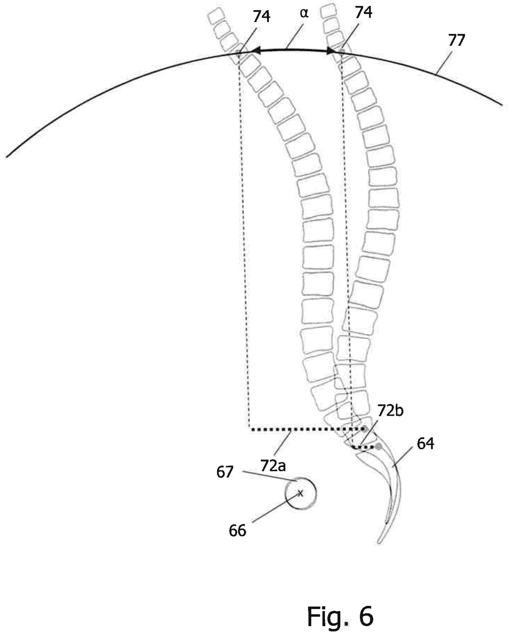

Details of the technique disclosed herein, including the method of Fig. 2, will now be explained in other words while referring to Figs. 3a-6. Figs. 3a and 3b illustrate various spinal balance parameters. Fig. 4a illustrates an exemplary medical image 49 (e.g., obtained in step 202) and Fig. 4b illustrates an exemplary second medical image 53 (e.g., obtained in step 203) in accordance with the present disclosure. Fig. 5a illustrates poses of the at least two bones 16, 18 as indicated by the alignment data and may be part of a navigation view 55 as triggered to be output in step 224. Fig. 5b illustrates an exemplary modified medical image 57 that may be used as the prediction image. Fig. 6 illustrates a rotation of a modified medical image that may be applies in step 212.

Generally speaking, the technique disclosed herein may allow assessment of the spinal balance based on measured (e.g., tracked or imaged) vertebral poses as indicated by the alignment data. The measurements are combined with information derived from the at least one medical image (e.g., a pre-operative weight bearing image such as a standing X-ray), and the spinal shape is predicted in the form of at least one prediction image (e.g., indicating a predicted upright posture of the spine). Spinal balance parameters for assessment of the spinal balance may be visualized in real time.

Although some planning tools allow assessment of a spinal shape using a medical image, these tools cannot predict a spinal posture based on current vertebral poses. Also, reliable intra-operative measurements of the current spinal shape that allow to compare the current situation intuitively with a planned target are not available. If a plan was made, Fluro shots may be performed to verify that the part of the spine that is immobilized by fusing vertebrae together matches the surgical plan. Manual measurements in the Fluro shot as well as in a surgical plan may need to be made to verify that the plan is complied with. Such a procedure may expose the patient with a large amount of X-rays and may be time consuming. Also, if a situation differs from the surgical plan, current solutions do not provide a user with a prediction of spinal balance parameters such as pelvic tilt, sacral slope or the sagittal vertical axis.

The technique disclosed herein allows estimating spinal balancing parameters valid in the standing posture based on intra-operative measurements in the supine patient. The vertebral poses may be captured by the tracking system 10 by tracking bone trackers 34, 36 associated with respective vertebrae 16, 18. These tracked poses are used to modify the medical image (e.g., a pre-operative weightbearing image such as a standing X-ray), for example by dividing the medical image into a plurality of image portions and inserting wedges between the image portions to modify the medical image and, optionally, rotating the modified image for simulating the Pelvic Tilt that is assumed for the patient. A surgeon can include own assumptions by interacting with the system, which may be referred to as "on-the-fly planning". Global balancing and spinopelvic parameters may be instantly computed and displayed based on the prediction image. The prediction image may be (e.g., periodically) updated based on the (e.g., up-to-date) alignment data. In this case, the prediction image may be referred to as a "live image". A visualization of the prediction image showing the estimated spinal shape in the upright position, including parameters, color coded according to the assumed outcome, may allow the surgeon to instantly assess the current situation.

The method may reduce the required number of Fluro shots and decrease radiation. It may increase the efficiency and effectivity of intervention by dynamically updating the prediction image based on current poses (e.g., up-to-date alignment data) and visualizing the estimated spinal alignment under a weight bearing condition by displaying the prediction image. It may allow to assess the alignment in the standing posture during an intervention without the need of performing a preoperative planning.

The disclosed technique generally allows intra-operatively deriving estimated global balancing parameters of the spine (e.g., the at least one spinal balance parameter), valid in the standing posture, based on intra-operative measurements in the supine patient (e.g., based on the prediction image that is determined based on the alignment data).

Intra-operative poses of vertebrae 16, 18 can be captured by the tracking system 10 and can be used to assess changes on the medical image (e.g., a pre-operative weightbearing image such as a standing X-ray). Spinopelvic parameters (e.g., Pelvic Incidence), may be derived from the medical image to classify the patient case. For example, the patient-specific pelvic tilt may be derived from the medical image and may be considered for keeping the trunk of the patient as upright as possible.

Depending on the (e.g., inter-operative) poses of vertebrae indicated by the alignment data, the medical image is modified, for example by inserting adequate wedges between image portions of said image. The surgeon may provide user input to change the Pelvic Tilt, or the PT may be adjusted automatically to a predefined, optionally patient-specific value. After the modification of the medical image, global balancing parameters and spinopelvic parameters may be derived from the modified medical image (e.g., used as the prediction image) and displayed, for example together with a literature-based normative range supporting a surgeon in assessing the outcome of the intervention.

In the technique disclosed herein, it may be assumed that after aligning the patient's spine (e.g., in the lumbar region) the spinal shape stays the same for the vertebrae (e.g., 20-26) which poses are not indicated by the alignment data which poses are, thus, not directly considered for modifying the medical image. It may further be assumed that a normative range or expected spinal parameters valid for the standing posture can expressed given a PI and other parameters like patient age and BMI.

There are several well-known parameters for assessing spinal alignment. These parameters are generally referred to as spinal balance parameters herein and may be measured in a weightbearing image, e.g., in the medical image, the modified medical image or the prediction image. Such parameters include pelvic parameters. Examples of pelvic parameters include Pelvic Incidence (PI), Pelvic Tilt (PT), Sacral Slope (SS) and the T1 pelvic angle (TPA). Pelvic Incidence is a fixed parameter for an individual that is providing an anatomical characterization of the pelvis. The ideal and the normative range of PT and SS depend on the PI. When the PI is measured, the normative range and the ideal value of the PT and SS can be computed, for example further based on patient-specific information such as age or BMI. As another spinal balance parameter, global balancing parameters like the Sagittal Vertical Axis (SVA) may be used. It may generally be desired to minimize the SVA during a spinal procedure as a value of the SVA close to zero is associated with a straight gaze and a physiological standing posture. Spinal balance parameters may include one or more of an axial, a coronal and a sagittal Cobb Angle (CA). Further spinal balance parameters derivable from the medical image, the modified medical image and/or the prediction image may be used in alternative or in addition to the aforementioned PI, PT, SS, TPA, SVA, CA.

With reference to Fig. 3a and 3b, it is apparent that the Sacral Slope, SS, 58, the Pelvic Incidence, PI, 60 and the Pelvic Tilt, PT, 62 are defined by the relative pose of the patient's pelvis (e.g., indicated by a pose of the sacrum 64) and a center 66 of the femoral head 67. Pelvic Incidence 60 is generally defined as the angle between a line 70 perpendicular to the sacral endplate at its midpoint 71 extending caudally and a line 73 joining the midpoint of the sacral endplate to the hip axis corresponding to the center of the femoral head 66. The Sacral Slope 58 is generally defined as the angle formed by a line 75 parallel to the sacral endplate and a horizontal line 77. The Pelvic Tilt 62 is generally defined as the angle formed by a vertical line 79 passing through the hip axis at 66 and the line 73. The Sagittal Vertical Axis, SVA, 72 is further defined by a relative pose between the most posterosuperior corner of the S1, designated with reference numeral 68, and the seventh cervical vertebra C7, designated with reference numeral 74. The T1 pelvic angle TPA 76 is defined as the angle between the line 73 and a line interconnecting the center of C7 and the center 66 of the femoral head 67.

An increased PT, in other words a backward rotation of the pelvis around the femur axis at 66, is a compensatory mechanism that allows the patient to achieve a lower value for the SVA in order to achieve a straight physiological standing posture. Too much pelvic tilt may not be energy efficient and be correlated with increased pain and disability. A preferred PT may be provided which leads in combination with an appropriate amount of Lordosis and/or Kyphosis correction to a physiological upright posture.

A surgeon might choose a cage with a specific lordotic angle in a lumbar disc space, resulting in a modified medical image with accordingly changed poses of the vertebrae separated by the chosen cage. The Pelvic Tilt of the modified medical image may be changed by rotating said image around the femur axis at 66 so that Pelvic Tilt and SVA are in a predefined normative range. As the Pelvic Tilt depends many factors like the muscle tone, bony morphology, pain and altered mobility, a surgeon may select for the specific patient case which Pelvic Tilt, and also which SVA is appropriate for the patient, even when literature based normative ranges and averages can provide indications.

A workflow in accordance with the present disclosure may comprise one or more of the following:
1. Acquire medical image data to be obtained in step 202, by capturing the (e.g., preoperative and/or weightbearing) medical image 49 such as a standing X-ray of the patient's body 28;
2. Input patient-specific information to be obtained in step 214 (e.g., age, BMI, weight etc.), that may be used for assessing postoperative alignment targets;
3. Automatic identification of landmarks in the medical image 49 for deriving at least one spinal balance parameter (e.g., spinopelvic and/or global balancing parameters) and, optionally, for registering the medical image;
4. Confirm the identified landmarks by a surgeon;
5. Acquire the second (e.g., intraoperative) medical image 53 to be obtained in step 203, the second medical image (e.g., a CT scan) being for example used for generating the navigation view to be triggered to be displayed in step 224;
6. Segment the at least two vertebrae 16, 18 in the second medical image 53 and identify the endplates of the segmented vertebrae 16, 18 which vertebrae are to be immobilized relative to one another; optional segmentation and endplate identification of other vertebrae 20-26; optional identification of landmarks for measuring spinopelvic and global balancing parameter(s);
7. Confirm segmentation, endplates, vertebral labels and/or landmarks in the second medical image 53 by a surgeon;
8. Patient registration: Establish a mapping, also referred to as transformation, of the second medical image 53 to the tracker(s) 34, 36 having fixed spatial relationships to the segmented at least two vertebrae 16, 18; establishing a mapping from the (e.g., intraoperative) second medical image 53 to the (e.g., preoperative) medical image 49 obtained in step 202;
9. Display a navigation view 55 to inform a surgeon on a relative pose between tracked instrument 30 and the tracked vertebrae 16, 18;
10. Simulation of upright posture: Modification of (e.g., weightbearing) medical image 49 in step 208 and, optionally, step 112 (e.g., rotation around femur axis to a pre-defined or re-calculated Pelvic Tilt), to obtain modified medical image 57, based on alignment data obtained in step 204 that may be based on tracking data obtained from the tracking system 10 tracking the trackers 34, 36;
11. Re-Calculation of spinopelvic and global balancing parameters based on the modified medical image 57 in step 216;
12. Outcome Visualization as consequence to triggering step 220 (e.g., display of the modified weightbearing image 57, the parameters derived therefrom and/or a normative range for one or more of the derived parameter(s));
13. Optional assessment of current spinal alignment by a surgeon based on the visualization;
14. Optional surgeon interaction with system (e.g., adjusting pelvic tilt in modified image by applying rotational transformation in step 212; 11. to 13. may then be repeated based on the rotated modified medical image);
15. Further spinal alignment, if needed, resulting in updated alignment data and/or additional rotation to be applied to the modified medical image 57; potentially followed by a repetition of 10. to 14.

As explained above, the present technique relies on obtaining the image data including the medical image 49 (step 202), obtaining alignment data (step 204), for example based on intra-operative measurements, and, as an option, obtaining patient-specific information (step 214) like patient age or patient BMI, that may be used for a formalization or accurate regression of postoperative alignment targets.

Based on the input obtained in steps 202, 204 and, optionally, 206, the at least one spinal balance parameter (e.g., including one or more spinopelvic parameters and/or one or more global balancing parameters) may be estimated by deriving the same from the modified medical image 57 and/or the prediction image (step 216). Normative ranges of the at least one spinal balance parameter may be determined based on the patient-specific information obtained in step 214.

The medical image 49 obtained in step 202 (e.g., a weightbearing image) is needed as input that contains representations of a plurality of bones 16-26. In other words, the medical image 49 depicts anatomical structures of the patient's body 28 that may be utilized for measuring parameters of interest which allow assessment of the spinal shape. The medical image 49 may also include indications about the patient orientation, enabling identification of a horizontal and vertical direction (e.g., a gravity line) relative to the medical image 49. The medical image 49 may be distortion-free. In other words, the medical image 49 may preserve anatomical lengths and angles to allow measurements of the at least one spinal balance parameter.

A lateral X-ray may be used as the medical image 49. Such an image allows for measuring sagittal alignment. Alternatively, or in addition, an AP image may be used to measure coronal balance. The technique disclosed herein is not limited to a 2D image 49, nor to sagittal aspects.

The (e.g., pre-operative) medical image 49 may be acquired in a (e.g., standardized) upright position in a relaxed body posture. Thus, the medical image 49 may be referred to as standing X-ray or weight-bearing X-ray. In this medical image 49, one or more landmarks may be identified automatically or manually to calculate spinal parameters of interest. These anatomical landmarks may include one or more heads of the patient's femoral bones, the S1 endplate (e.g., including the most posterosuperior corner of the S1), the midpoint of the vertebra C7, endplates of vertebrae of interest, landmarks for registration (e.g., postero- and anterosuperior points of endplates).

The one or more anatomical landmarks may be identified automatically, for example using a trained machine learning model (e.g., a Deep Learning based automatic identification of these landmarks). The landmark identification may be automatically triggered when the medical image 49 is obtained in step 202. A manual verification step may follow where the surgeons confirms that the automatically identified landmarks are valid. A similar landmark identification procedure may be performed for identifying landmark(s) in the modified medical image 57 and/or the prediction image, for example in step 218.

The alignment data indicative of the poses of the at least two vertebrae 16, 18, may be obtained from the tracking system 10. As explained above, tracking system 10 may be an optical tracking system. Alternatively, the tracking system 10 may be embodied as an electromagnetic, EM, tracking system comprising a field generator. The trackers 32, 34 may be active or passive optical trackers or EM-trackers. The trackers 32, 34 may be arranged in fixed spatial poses relative to the respective vertebra of interest 16, 18. Each vertebra 16, 18, whose pose (e.g., position and orientation) may be needed for a precise alignment when immobilizing portions of the spine, may be provided with a respective tracker. In case an interbody implant 56 is to be inserted into a disc space, the adjacent vertebrae 16, 18 may be provided with respective trackers. Vertebrae 20-26 which poses are not indicated by the alignment data may be treated as constant. In other words, their relative poses to the closest vertebra of interest 16, 18 in the standing posture may be assumed to remain constant.

Based on the alignment data, it is possible to determine angles between endplates of the vertebrae 16, 18. To measure the lordosis or kyphosis, planes approximating segmented endplates of the vertebrae may be projected into the sagittal plane, resulting in lines 45, 47 as illustrated in Fig. 5a. The angle between these lines 45, 47 expresses the lordosis or kyphosis. For example, for the vertebrae of interest 16, 18 the superior endplates can be chosen. The overall lumbar lordosis can be defined as the angle between the projected endplate lines 45, 47 through the sacrum endplate and/or the inferior endplate of L1.

Optionally, patient-specific information that influences the spinal shape and that cannot be computed directly based on the medical image 49, the modified medical image 57 or the prediction image, may be obtained in step 214. This information may include an amount of Pelvic Tilt that is assumed for the spinal alignment. Although a PT value from a predefined normative range may be automatically suggested, manual adjustment and confirmation by the surgeon may be performed, for example by applying a corresponding rotation to the modified medical image 57 in step 212 based on the patient-specific information and, optionally, based on further user input.

Once the medical image 49 and the second medical image 53 are obtained (steps 202, 203), the images 49, 53 may be registered to one another using of several image registration techniques known to those skilled in the art. This allows transferring poses from the second medical image 53 to the medical image 49 and vice versa (e.g., in the tracking coordinate system). There are several ways of registering the medical image 49 to the second medical image 53. A viewing direction may be derived from the medical image 49 and compared with a corresponding viewing direction of the second medical image 53 to obtain the registration. One way to achieve this is to generate digitally reconstructed radiographs, DRRs, out of a 3D image set forming the second medical image 53, from different perspectives, and finding a DRR that best matches the medical image 49. Following this approach, corresponding landmarks can be identified accurately. Determining the registration may comprise computing a transformation matrix that transforms points from the medical image 49 to the second medical image 53. These points may correspond to positions of landmarks identified in the respective images.

The transformation matrix may be determined by measuring at least three corresponding points in both the medical image 49 and the second medical image 53, and determining a rigid transformation matrix between these points.

The medical image 49 is modified in step 208 based on the alignment data. This may include applying current poses of the vertebrae of interest 16, 18 to the standing X-ray 49 and (re-)calculating the at least one spinal balance parameter while keeping constant the Pelvic Tilt (e.g., without performing step 212). Thereby, the current poses of the vertebrae of interest 16, 18 can be introduced into the weightbearing image 49. This may involve dividing the medical image 49 into image portions 41, 43 (step 210), for example by cutting it along a line 59 approximating the superior endplate of the inferior vertebra 16 as represented by the medical image 49, which interior vertebra 16 is to be joined to a superior vertebra 18, and changing a pose of the superior image portion 41 such that the pose of the superior vertebra 18 included in the superior image portion 41 matches the current pose of the vertebra 18 relative to the current pose of the vertebra 16 as indicated by the alignment data (e.g., at least by exhibiting a corresponding angle between the lines approximating the vertebral endplates of the vertebra of interest 16, 18). One may say that this approach creates a wedge 44 (e.g., +5° lordosis between L4 and L5) in the modified medical image 57 that is not present in the medical image 49.

Lordosis or kyphosis may be determined based poses of the vertebra of interest 16, 18, in particular based on the relative poses of the superior endplates of the superior and inferior vertebrae 18, 16 involved in the intended spinal fusion procedure. For example, for a fusion of vertebrae L4 (e.g., 16) and L5 (e.g., 18), points 48 and 52 on the superior endplate of L4 may be identified in the second medical image 55, as well as points 46 and 50 on the superior endplate of L5 in the second medical image 55. The respective set of points 48, 52 and 46, 50 defines the pose of the respective vertebra 16, 18. Thus, these points may be used to modify the medical image 49, thereby creating wedge 44. In particular, the medical image 49 may be divided into the plurality of image portions 41, 43 in step 210 by cutting the medical image 49 into a superior image portion 41 and an inferior image portion 43 along line 59 intersecting the corresponding endplate points 46, 50 of the inferior vertebra 16 in the medical image 49. The modified medical image 57 may be triggered to be displayed. In case no additional rotation is to be applied as part of step 212, the modified medical image corresponds to the prediction image.

For assessing the estimated spinal shape, a surgeon may be provided with one or more of the following interaction possibilities:

### (i) Manual adjustment of the Pelvic Tilt.

The surgeon may provide user input defining an intended pelvic tilt. This user input may be part of the information obtained in step 214. The modified medical image 47 may then be rotated accordingly around the femur axis in step 212. The at least one spinal balance parameter may then be (re-)calculated based on the rotated modified medical image 57, which may be referred to as prediction image. The (e.g., updated) spinal balance parameter(s) may be displayed in step 220 together with the normative range of the parameter(s), the normative range being optionally determined based on the patient-specific information obtained in step 214, to assist the surgeon in predicting the outcome.

### (ii) Automatic adjustment of Pelvic Tilt and/or SVA

An automatic Pelvic Tilt and/or SVA adjustment may be performed to bring the respective spinal balance parameter into the normative range, or to a predefined ideal (e.g., patient-specific) value. Various options for such an automatic adjustment are possible.

In a first option, the PT is automatically adjusted by applying a rotational transformation to the modified medical image 57 such that the modified medical image 57 is rotated around the femur axis at 66 so that the PT reaches the closest value of its normative range.

In a second option, the PT is automatically adjusted by applying a rotational transformation to the modified medical image 57 such that the modified medical image 57 is rotated around the femur axis at 66 so that the PT reaches an average PT value (e.g., a value in the middle of the normative range).

In a third option, the SVA is automatically adjusted by applying a rotational transformation to the modified medical image 57 such that the modified medical image 57 is rotated around the femur axis at 66 so that the SVA reaches the closest value of its normative range.

In a fourth option, the PT is automatically adjusted by applying a rotational transformation to the modified medical image 57 such that the modified medical image 57 is rotated around the femur axis at 66 so that the SVA reaches an average SVA value (e.g., a value in the middle of the normative range).

Two of the above options may be combined. That is, a rotation may be applied such that both the SVA and the PT fulfil predefined criteria (e.g., fall into the normative range). In this case, both the Pelvic Tilt and the SVA may be optimized. This may be a preferred option when no additional background knowledge provides an indication for one of the above first to fourth options.

To optimize both, the PT and the SVA, an objective function may be used. As SVA depends on PT, there is just one degree of freedom, which is the PT, or in other words a rotation about an angle α around the center 66 of the femur head 67, as indicated in Fig. 6. When increasing the Pelvic Tilt, C7 and its center are moving clockwise on a circle 77 around the femur axis point 66 which decreases the SVA from 72a to 72b. Taking the geometric considerations of Figs. 3a and 6 into account, SVA depends on the amount of Pelvic Tilt and on two points, the center of the vertebra C7 and the midpoint 71 of the S1 endplate. As for a fixed spinal shape the relation of these both points is constant, the SVA has just one degree of freedom, namely the rotation angle α and therefore the PT.

A function SVA(t) may be used that describes the SVA given a PT with a value of t. Assume p(t) is a probability distribution of the PT t and s(t) is a probability distribution for the SVA which can be expressed as SVA(t). Even without knowing the exact probability distributions, a simplified model can be used representing these functions, for example as triangular functions with a maximum value of 1 at the average and a support defined by the normative range. Different functions may be used depending on measured patient-specific spinal balance parameters such as the PI and/or the patient-specific information such as the patient's age. The functions p and s can also be seen as functions describing the goodness of the PT respectively the SVA. An objective function can be created combining both with a t-Norm or s-Norm, for example by a multiplication of both. Thus, the optimal PT to be applies by the rotation performed in step 212 may be considered to correspond to t_ideal= argmaxt p(t) * s(t).

Using this automatic optimization of the PT and SVA makes it possible to get instant feedback (e.g., due to the display caused by step 220) about the alignment during a surgery without any further action. The interaction option (e.g., defining the rotation around the femur axis point 66, e.g., via selection of different automatic adjustment buttons) may be selected by the user, allowing a surgeon to control the PT assumed for the patient and to decide which exact values for SVA and PT are expected.

The instant feedback provided by displaying the modified medical image 57 and/or prediction image in combination with the spinal alignment parameters derived therefrom may be visualized on a scale including color coding, e.g. green around an ideal value, yellow for a normative range and red for values outside the normative range that predict disability, allowing a surgeon to assess whether the achieved lordosis or kyphosis is acceptable. As mentioned above, visualized parameters may include, but are not limited to PI, PT, SS, SVA, PI-LL mismatch.

As explained above, poses of the vertebrae 16, 18 are directly used for the modification of the medical image 49 and the parameter calculations, whereas non-tracked vertebrae may be assumed as constant. Their poses may be derived from the medical image 49 or from the second medical image 53.

The technique disclosed herein is not limited to sagittal balancing. The coronal spinal balance can be evaluated in a similar manner, for example when using a standing AP X-ray image as the medical image 49. It is also possible to assess axial spinal balance. In this case, the axial angle of the vertebrae 16, 18 and in particular of a most superior and most inferior of tracked vertebra may be shown to assess whether the target of a 0° axial angle is achieved when fusing the vertebrae 16, 18.

Further details of the at least one spinal balance parameter will now be explained.

One of the most important radiographic parameter is Pelvic Incidence (PI), which is considered a key factor for managing spinal balance. PT is significantly increased in patients with degenerative spondylolisthesis and dysplastic spondylolisthesis, and the relative increase in pelvic incidence correlates directly with the severity of the slip

When the Pelvic Incidence is given, just one of the parameters Pelvic Tilt or Sacral Slope may need to be measured as the equation SS +PT=PI is true for a given individual in the standing patient's posture. For assessing the spinal curvature, Sacral Slope or Pelvic Tilt may need to be measured in the standing patient's posture in a relaxed state.

Pelvic Incidence is generally fixed and may not change for each individual. It may be referred to as a fixed angle that becomes set at the end of growth. In contrast, Pelvic Tilt and Sacral Slope are variable. The values of PT and SS are changing when the patient is tilting the pelvis by rotating it around the femoral axis which is possible up to a certain limit. After a correction surgery, e.g. a spinal fusion, the values for both parameters PT, SS are in generally different in the standing patient's posture compared to the state before the surgery.

In contrast to the other parameters, the PI may not be used for assessing a spinal curvature, as there may not be predefined "good" or "bad" values for the PI available. However, determining the PI may be beneficial, as it provides an anatomical characterization of the pelvis and the ideal and the normative range of other parameters may depend on it.

An average value for the PI may be assumed to be 55° ± 10°. PI may be assumed to increase with age according to PI = 44.3 + 0.2 * age.

Along with pelvic parameters, sagittal vertical axis (SVA) is one of the most important radiographic parameters to assess global balance. SVA is generally defined as a distance from a plumb line dropped from the C7 vertebra and the posterosuperior corner 68 of the sacrum S1 64.

A small Pelvic Incidence (PI) means that the sacrum is almost vertical and the L5 endplate is almost horizontal. So a small Lumbar Lordosis (LL) is generally sufficient for an upright position, while for a large PI, the sacrum is oriented more horizontal and a large lumbar lordosis is required to achieve the upright position. Thus, the lordosis depends on the Pelvic Incidence of the specific patient. A small PI-LL mismatch can be used as a simplified way to assess the degree of realignment necessary and to approximate an ideal lordosis for a patient. A value of PI-LL close to zero may be assumed to be beneficial, as higher values may be associated with higher level of pain and disability. An ideal PI-LL may be assumed to lie below 10°. PI-LL ≥11° may lead to disability. However, in some conditions slightly higher values of PI-LL might be ideal (e.g., values between 10° and 20° in ADS patients after long posterior instrumentation and fusion).

Additional parameters like the patient's age can provide a more accurate definition of the LL. For example, the lumbar lordosis between L1 and S1 may be expressed by LL = 0.5 PI + 28.5, and when the age of the patient is considered too, a more accurate formula may be LL = 31.1 - 0.15*age + 0.58*PI. Another example for a formula for the lumbar lordosis is LL = 32.9 + 0.60PI - 0.23age.

Before finally fixating the vertebrae 16, 18, a intraoperative real-time measurement of the lumbar lordosis can help to estimate whether the current lumbar lordosis would lead to a balanced spine. For the PI-LL mismatch, just the lumbar lordosis and the Pelvic Incidence may need to be regarded.

The PI-LL mismatch is a simple measurement that does not include the pelvic tilt compensation mechanism, as it can be measured directly when the L1 vertebra and the sacrum are tracked, or it can be derived straight forward as explained below, when some vertebrae are tracked and other are assumed as constant. No interaction, e.g. for estimating the pelvic tilt, may be needed for deriving the PI-LL mismatch.

The outcome regarding sagittal alignment may not only depend on the lumbar lordosis for a classification according to a given Pelvic Incidence, even if the PI-LL mismatch already provides useful information. The spinal shape of the thoracic vertebrae and the amount of patient specific compensatory mechanism of the pelvic tilt may also help assessing the alignment. When Sagittal Vertical Axis and the Pelvic Tilt are outside the normative range, in general, the patient cannot adopt to a balanced spine in a relaxed standing patient's posture. When these parameters are included in the assessment, a more case-specific and differentiated prediction of the outcome of a spinal correction is possible.

Restoring global alignment expressed in a value for the Sagittal Vertical Axis (SVA) close to zero facilitates straight gaze and the achievement of a physiological standing posture. The average SVA may be assumed to be SVA_average = -3.1 + 0.08*age (e.g., with R = 0.3906, p < 0.0001). It may be desirable to obtain a postoperative SVA of less than 50 mm. An SVA ≥47 mm may be assumed to lead to severe disability.

The Body Mass Index (BMI) can influence the alignment targets. For example, for patients with a high BMI, age-adjusted alignment targets seem to be less substantial. After surgery, obese patients are often under corrected and have a higher global sagittal angle (e.g., obese: >6.5; normal: 3.40).

When the Pelvic Incidence is measured, also a normative range for the Pelvic Tilt can be expressed. Pelvic tilt is a compensatory mechanism that allows patients to achieve sagittal balance when the lumbar lordosis is decreased. A restoration of the lumbar lordosis allows the pelvis to rotate forward to return to the normal theoretical value of PT and SS. For example, PT may be assumed to be PT = 0.448*PI - 11.33 or PT = 0.44*PI - 11.5.

Pelvic retroversion, which is a backward rotation of the pelvis, is a compensatory mechanism to lower the SVA and to "bring back" the spine into a vertical position. From a physiologic point of view, an increase in PT is not energy efficient, and correlates with increased pain and disability. A small amount of retroversion and a changed spinal shape may be sufficient to achieve a straight gaze and a upright physiological standing posture (low SVA).

It may be assumed that the PT increases with age according to the regression equation PT = 3.0 + 0.2*age (e.g., with R = 0.3146, p = 0.0003).

Generally speaking, for a spinal fusion procedure, the surgeon needs to decide before fixating vertebra to one another whether the changed spinal shape will lead to a physiological standing posture. The technique disclosed herein supports the surgeon accordingly, leading to the advantages detailed above. Modifications of the system and method disclosed herein are possible, for example by using an electromagnetic tracking system instead of an optical tracking system. As another modification example, instead of using a LAT image as the medical image 49, an AP image may be used. Further modifications and advantages of the present technique will be apparent to those skilled in the art.

## Claims

1. A method for determining at least one prediction image (57) based on at least one medical image (49) of a patient, the method being performed by at least one processor (4) and comprising:
obtaining (202) image data including at least one medical image (49) comprising representations of a plurality of bones (16-26) of a patient's body (28) in a first body posture;
obtaining (204) alignment data indicative of an alignment of at least two (16, 18) of the plurality of bones (16-26) of the patient's body (28) in a second body posture differing from the first body posture;
determining (206), based on the image data and the alignment data, at least one prediction image (57) comprising representations of the plurality of bones (16-26) of the patient's body (18) in the first body posture, wherein determining the at least one prediction image (57) comprises modifying the at least one medical image (49) based on the alignment data such that poses of the representations of the at least two bones (16, 18) after the modification comply with the alignment of the at least two bones (16, 18) indicated by the alignment data; and
triggering display of one or more of the at least one prediction image (57) and/or of information (58, 60, 62, 72) derived from one or more of the at least one prediction image.

2. The method of claim 1, wherein
the at least two bones (16, 18) are in a weight bearing state when the patient's body (28) is in the first body posture and/or in a non-weight bearing state when the patient's body (28) is in the second body posture.

3. The method of claim 1 or 2, wherein
the first body posture corresponds to a standing posture and/or the second body posture corresponds to a lying posture.

4. The method of any one of claims 1 to 3, wherein
modifying the at least one medical image (49) comprises dividing (210) the at least one medical image (49) into a plurality of image portions (41, 43) and changing a pose of at least one of the plurality of image portions (43) relative to one or more other ones (41) of the plurality of image portions (41, 43).

5. The method of claim 4, wherein
each of the plurality of image portions (41, 43) contains a representation of a respective different one of the at least two (16, 18) of the plurality of bones (16-26).

6. The method of claim 4 or 5, wherein
the at least one medical image (49) is divided at least along a line (59) or plane associated with a surface of one of the at least two bones (16, 18).

7. The method of any one of claims 1 to 6, wherein
the at least two bones (16, 18) correspond to different vertebrae.

8. The method of claims 6 and 7, wherein
the line (59) or plane is associated with an endplate of one of the different vertebrae (16) facing toward another one of the different vertebrae (18).

9. The method of any one of claims 1 to 8, wherein
determining the prediction image (57) comprises applying (212) a rotational transformation to the modified at least one medical image to adjust at least one spinal balance parameter of the patient's spine derived from the modified at least one medical image.

10. The method of claim 9, further comprising:
obtaining (214) patient-specific information indicative of one or more of an age of the patient, a gender of the patient, a height of the patient, a weight of the patient and/or a Body Mass Index, BMI, of the patient,
wherein the rotational transformation is determined based on the patient-specific information.

11. The method of claim 9 or 10, wherein
the rotational transformation is determined such that by applying the rotational transformation to the modified at least one medical image (49), the modified at least one medical image is rotated around a femoral rotation axis.

12. The method of any one of claims 1 to 11, further comprising:
deriving (216), as the information, at least one spinal balance parameter from one or more of the at least one prediction image (57), wherein, optionally,
deriving the at least one spinal balance parameter comprises identifying at least one anatomical landmark (64-68) in the one or more of the at least one prediction image (57) and determining the at least one spinal balance parameter based on a pose of the identified at least one anatomical landmark (64-68).

13. The method of any one of claims 9 to 12, wherein the at least one spinal balance parameter comprises one or more of the following:
Pelvic Incidence, PI;
Pelvic Tilt, PT;
T1 pelvic angle, TPA;
Cobb angle;
Sacral Slope, SS;
Sagittal Vertical Axis, SVA.

14. The method of any one of claims 1 to 13, wherein
the at least one medical image (49) comprises a pre-operative lateral, LAT, X-ray image and/or an anterior-posterior, AP, X-ray image.

15. The method of any one of claims 1 to 14, wherein
the alignment of the at least two bones (16, 18) comprises at least two poses selected from (i) a tracked pose of one or more of the at least two bones (16, 18) of the patient's body (28) in the second body posture and (ii) an imaged pose of one or more of the at least two bones (16, 18) of the patient's body (28) in the second body posture.

16. The method of claim 15, further comprising:
obtaining (203) at least one second medical image (53) comprising representations of the at least two bones (16, 18) of the patient's body (28) in the second body posture;
obtaining tracking data (222) indicative of a tracked pose of a surgical instrument (30); and
triggering display of a navigation view (55) that is based on one or more of the at least one second medical image (53) and based on the tracking data and visualizes a tracked pose of the surgical instrument (30) relative to the at least two bones (16, 18) of the patient's body (28) in the second body posture.

17. A system (100) comprising at least one processor (4) configured to perform the method according to any one of claims 1 to 16.

18. The system (100) of claim 17, further comprising at least one of the following entities:
a tracking system (10) configured to acquire a tracked track pose of one or more of the at least two bones;
an image acquisition system (14) configured to acquire a medical image of one or more bones (16-26) of the patient's body (28);
a display unit (42) configured to display the one or more of the at least one prediction image (57) and/or the information derived from the one or more of the at least one prediction image (57) upon being triggered to display said image(s) and/or information.

19. A computer program comprising instructions which, when executed on at least one processor (4), cause the at least one processor (4) to carry out the method according to any one of claims 1 to 16, the computer program being optionally carried by at least one carrier such as a non-transitory computer-readable storage medium (6).
